# EUROPEAN PATENT APPLICATION

(11) **EP 0 886 140 A1**
(43) Date of publication of application: **23.12.1998**
(21) Application number: 98304518.8
(22) Date of filing: 08.06.1998
(51) Int. Cl.: G01N 33/50, G01N 33/573, G01N 33/74

(54) **Chronic wound model**

(30) Priority: 09.06.1997 US 49161 P
(71) Applicant: Ethicon, Inc., Somerville, New Jersey 08876-0151 (US)
(72) Inventor: Niciporciukas, Maria C., Lawrenceville, NJ 08648 (US)
(74) Representative: Fisher, Adrian John

(57) **Abstract**

A novel wound healing model and method for studying the pathophysiology of chronic wounds is disclosed. Diabetes is induced in a pig via injection of streptozotocin. Levels of collagenase and elastase are increased in the diabetic pig wounds versus non-diabetic pigs thereby simulating conditions in a chronic wound.

## Description

### Field of the Invention

This invention relates to a model for the study of healing of chronic wounds. Specifically, the invention relates to the use of a diabetic pig as a model for the study of the pathophysiology of chronic wounds in order to study the effect of wound healing agents and other compounds.

### Background of the Invention

Wound fluid reflects the environment of the wounds from which it is collected and thus may offer clues to the events occurring during wound repair or its failure. Although no specific enzyme has been associated with the impaired wound healing detected in human diabetic patients, wound fluid obtained from chronic wounds from different etiologies has been found to have a high level of proteases (Wysocki AB, Staiano-Coico L, Grinnel F: "Wound fluid from chronic leg ulcers contains elevated levels of metalloproteases MMP-2 and MMP-9". *J. Invest. Dermatol.*, 101:743-8, 1993; Yager DR, Chen SM, Ward SI, Olutoye OO, Diegelmann RF, Cohen IK: "Ability of chronic wound fluids to degrade peptide growth factors is associated with increased levels of elastase activity and diminished levels of proteinase inhibitors." *Wound Rep. Reg.*, 5:23-32, 1997; Yager DR, Zhang L-Y, Liang H-X, Diegelman RF, Cohen IK: "Wound fluids from human pressure ulcers contain elevated matrix metalloprotease level and activity compared to surgical wound fluids." *J. Invest. Dermatol.,* 197:743-8, 1996). The high levels of degradative enzymes are associated not only with the degradation of the extracellular matrix, but also with the inactivation of exogenously added growth factors.

The shortcomings of using rats or mice as species to study the wound healing process reside in the fact that the skin anatomy of these animals is quite different from that of human beings (Cohen & Mast, 1990; Kiristy & Lynch, 1994). Rodents are loosely skinned animals and their skin contains an attached muscle layer underneath, the panniculus carnosus, which is not present in humans (Richey et al., 1989; Kiristy & Lynch, 1993). As a result, these animals heal by wound contraction to a much larger degree as compared to tight skinned animals like humans and the wound healing results in rodents typically cannot be translated to humans (Kiristy & Lynch, 1994).

Similarly to humans, pigs are considered tight-skinned animals, and only 25% of total wound closure may be attributed to wound contraction (Kiristy & Lynch, 1993). However, although the structural, chemical and functional properties of pig skin compare well with those of the human skin (Forbes, 1969; Bissett & McBride, 1985), pre-clinical studies with this species have heretofore been performed only using normal-healing, non diabetic animals and no one appears to have studied the wound healing properties of a diabetic pig.

Granulation tissue is needed in human chronic wounds, not only to provide a fibrovascular support for the epidermis, but also to fill-in large defects like the ones detected in diabetic patients (LeGrand EK, Burke JF, Costa DE, Kiorpes TC: Dose responsive effects of PDGF-BB, PDGF-AA, EGF, and FGF on granulation tissue in a guinea pig partial thickness skin excision model." *Growth factors,* 8:307-14, 1993). Because of the characteristics of the pig skin, the wound healing process itself as well as the effect of growth promoting factors and/or modulators of the wound healing process can be studied more similarly to what happen in the human situation.

We have now successfully induced diabetes in the pig and have developed a novel, impaired wound model using swine as the species. The diabetic pig fulfill our needs for a model that shows impairment in granulation tissue formation. Wounds in this model exhibit increased collagenase and elastase levels similar to chronic wounds, whether in a diabetic or non-diabetic patient. Accordingly, the present invention provides a new and useful model and method for studying chronic wound healing and for evaluationg methods of treating chronic wounds.

The method also has utility in the study of diabetic wounds. A certain portion of the human population is suffering from diabetes, a metabolic disease which manifests itself by an impaired ability to digest sugars and predisposes human patients to the development of chronic wounds, or wounds that heal poorly or not at all. While a healthy person experiences wound healing through mainly granulation tissue formation and epithelialization, a diabetic patient's ability to heal by these parameters is impaired as a direct result of the metabolic abnormalities associated with the disease.

Chronic wound is a well established, but not fully understood complication of diabetes mellitus (Ahroni et al., 1993; Falanga et al., 1995). The complete understanding of the pathophysiology of diabetic chronic wounds, as in any other biological phenomenon, is dependent upon the use of animal models. The study of the delayed wound healing process associated with this metabolic disease has been performed in rodents, particularly in chemically-induced diabetic rats (Davidson et al., 1997) or in genetically mutant mice (Greenhalgh et al., 1990; Senter et al., 1995).

### Summary of the Invention

Our studies have found that a diabetic impaired wound model, using the pig as the species, can be used as a tool for understanding the pathophysiology of human chronic wounds as well as to assess the effect of growth promoting agents and/or modulators of the wound environment.

A method for the study of the pathophysiology of human chronic wounds according to the present invention comprises chemically inducing diabetes in a swine, creating at least one wound on the swine and analyzing exudate from the wound or wounds. Preferably, such analysis includes measurement of the levels of collagenase and elastase. To examine treatment methods for chronic wounds, the wound may be treated and the exudate from the treated wound analyzed. Preferably, the swine is selected from the group consisting of domestic pigs, Yucatan pigs, miniature pigs and any pig considered to have a tight skin.

### Brief Description Of The Drawings

FIG. 1 is a graph of a histological assessment of granulation tissue in diabetic x non-diabetic pig, 7 days post-wounding; and
FIG. 2 is a graph of collagenase-like activity in wound fluid collected from dressings placed on diabetic and non-diabetic pig wounds.

### Detailed Description Of The Invention

Pigs (*Sus scrofa*) are intravenously injected with a single dose of 150mg/kg of streptozotocin or streptozocin (STZ). Rapid administration of STZ is the best method to produce a distinct insulin-deficient diabetes in swine. STZ is quickly inactivated by the plasma pH, so it needs to be administered in a way that rapidly reaches the target organ, the pancreas. Other administration routes may not be as successful for diabetes induction in pigs, as described in Marshall M: "Induction of chronic diabetes by streptozotocin in the miniature pig." *Res*. *Exp. Med*. *175*:187-96, 1979.

Ahhough this is the preferred dose for a single application, STZ can be administered in the range of 85 to 200mg/kg. Also, two or more doses of STZ can be administered with the same purpose, 5 to 8 days apart, in the range of 30 to 100mg/kg. STZ is preferentially dissolved in sodium citrate buffer, at pH 4.5, but any other low pH buffer solution can be utilized.

The animals are starved for at lead 18 hours before experimental procedures and STZ injection. Alloxan or any other diabetic inducers can be used for the same purposes. Plasma glucose levels equal to or above 200mg/dl must be achieved to characterize the animal as diabetic. If such a glucose level is not achieved, the diabetic state is not clinically characterized and the animal should not be used for the purposes of this work. Conversely, plasma glucose levels should not exceed about 500mg/dl as the chances of survival of the animal become severely compromised.

The plasma glucose level is measured by collecting blood taken most commonly from the ear vein and by using glucose strips and a portable glucose meter (for example, One Touch Blood monitoring system, Lifescan, Milpitas, CA) or by any other biochemical methodology available (Wilson JD, Dhall DP, Simeonovic CJ, Lafferty KJ: "Induction and management of diabetes mellitus in the pig." *Aust. J*. *Exp. Biol. Med Sci.,* 64(Pt. 6):489-500, 1986).

Most preferably, full thickness excisions in the shape of 1.5 x 1.5cm squares and just dermis removed are created on the dorsal paravertebral skin of the animal using a scalpel blade. However, the wounds can range from about 0.5 x 0.5cm to about 5.0 x 5.0cm. The full-thickness excisions can also be made down to the muscle and/or have a circular, rectangular or a triangular shape.

Briefly for the surgical procedures, aseptic techniques should be followed during the surgery, wound assessment and dressing changes. One or multiple wounds can be made. Wounds can be made parallel to the spine or arranged in an alternate pattern. An acetate template can be used to aid in making the wounds to adequate size. A pair of scissors and/or a scalpel blade can be used to aid in the removal of skin and subcutaneous tissue. Bleeding can be stopped by sponge tamponade.

After wounding, each wound can be submitted to a treatment regimen. Test formulations and materials can be applied topically or injected. Secondary dressings can be applied to hold primary dressings in place. Other wound types can be performed. Preferably partial-thickness excisions, but also full-thickness incisions and/or thermal or chemical burns can be made.

Preferably a keratome but also a scalpel blade can be used to make the partial-thickness wounds. Full-thickness incisions can be made preferably with a scalpel blade. Heated devices (brass rods and/or electrical devices) can be used to inflict burn wounds. For excisional wounds or burns, the area/wound should not be smaller than about 0.25cm² or bigger than about 25cm².

The wounds are made most preferably 3 weeks after STZ or any other diabetes inducer but can also be performed as early as 5 days and up to 24 months post-STZ injection. Insulin in the range of 10 to 40 IU can be administered daily and up to 1 week before the wounding to ensure a good health state.

Wounds are left uncovered or are individually covered with a semi-occlusive polyurethane dressing such as BIOCLUSIVE™ for example. The wound healing process is evaluated at different time points after wounding. Granulation tissue formation is most preferably observed from 5 to 8 days post- wounding, but can be also be assessed up to 15 days post-wounding.

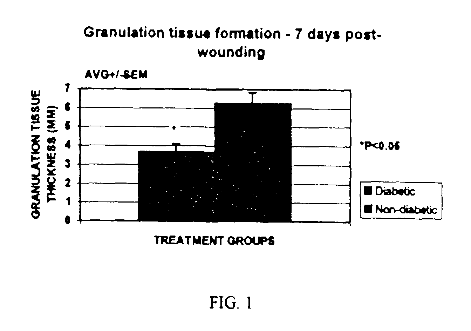

Diabetic pigs develop a dramatic, significant impairment in the wound healing response, particularly in granulation tissue formation (Figure 1). Visual and histological observations show a much reduced (approximately 50%) granulation tissue response in diabetic animals vs. normal (non-diabetic) animals when 7 day-old wounds are analyzed.

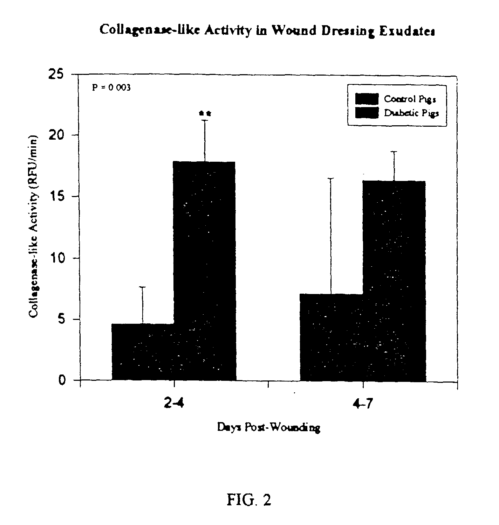

On the biochemical level, analysis of wound fluid collected from dressings placed over the wound bed and extracted by using a mild buffer (for example, 0.1M-Tris/HCI, pH 7.4 containing 0.1% Triton X-100) shows a 2 to 3-fold increase in the levels of metalloprotease activty (collagenase-like) in diabetic pigs as compared to non-diabetic animals (Figure 2). Also, elastase activity level is doubled in diabetic animals as compared to non-diabetic ones when tissue samples are collected from wounds and homogenized in a mild extraction buffer (data not shown). The level of metalloproteases in the wound fluid or tissue samples was analyzed as described below. Each class of enzyme listed above uses a different substrate for its activity, which is described for each methodology.

### Extraction of protein from dressings:

Wound fluid was extracted from the "release" dressings by incubating the material in a small volume of the wash buffer (1 ml; 0.1M-Tris/HCl, pH 7.4 containing 0.1% Triton X-100). This wash step was carried out at room temperature with shaking for approximately 2 hours, after which the solution was removed, aliquoted and stored frozen(-70°C). Once an aliquot was thawed it was tested immediately and any remaining sample was discarded due to the labile nature of the proteases and proteins present.

### Extraction of protein from tissue samples:

Tissue samples were analyzed for protease levels by homogenizing a 3mm punch biopsy of granulation tissue in a mild extraction buffer (2ml; 0.1M-Tris/HCl, pH 7.4 containing 0.1% Triton X-100). For comparison between samples the wet weight of each biopsy was measured prior to homogenization and the solubilized protein was recovered from this homogenate by a centrifugation step (approximately 6,000 x g - to remove tissue debris). This protein sample was aliquoted and stored frozen (-70°C) and as above, once thawed the remaining sample was discarded.

### Protein analysis:

A quantitative measure of the total protein extracted from either the dressings or tissue samples was determine using the Bradford assay. The Bradford assay has been implemented because it is simple, fast and has been optimized for use as a microtitre plate assay.

### Protease activity assays:

The active level of key proteases was determined by fluorimetric activity assays. These are rapid microtitre plate assays which quantify the level of active enzymes present in a biological sample and are based on the ability of the enzyme to specifically cleave a particular substrate which is mimicked by the labeled peptide.

Collagenase-like activity was measured using a succinyl-gly-pro-leu-gly-pro-7-amino-4-methyl coumarin substrate, by monitoring the release of 7-amino-4-methyl coumarin at 455nm (excitation at 383mn). The turnover of this substrate (used at a final concentration 50mM) was determined in a collagenase assay buffer - 40mM Tris/HCl, pH 7.4 containing 200mM NaCI and 10mM CaCl₂, with the reaction maintained at a constant temperature of 37°C.

While the invention has been particularly described in connection with specific embodiments thereof, it is to be understood that this is by way of illustration and not of limitation, and that the scope of the appended claims should be construed as broadly as the prior art will permit.

## Claims

1. A method for the study of the pathophysiology of human chronic wounds comprising chemically inducing diabetes in a swine, creating at least one wound on said swine and analyzing exudate from said at least one wound.

2. A method according to claim 1 wherein the analysis of the wound exudate includes measurement of the level of collagenase.

3. A method according to claim 1 wherein the analysis of the wound exudate includes measurement of the level of elastase.

4. A method according to claim 1 wherein the swine is selected from the group consisting of domestic pigs, Yucatan pigs, miniature pigs and any pig considered to have a tight skin.

5. A method according to claim 1 wherein the step of chemically inducing diabetes in the Swine includes the step of injecting the swine with streptozotocin.

6. A method according to claim 1 and further comprising the steps of treating at least one of the at least one wounds and analyzing exudate from said at least one of said at least one wound.
